# EUROPÄISCHE PATENTSCHRIFT

(11) **EP 1 831 273 B1**
(45) Veröffentlichungstag und Bekanntmachung des Hinweises auf die Patenterteilung: **21.05.2008**
(21) Anmeldenummer: 05850319.4
(22) Anmeldetag: 21.12.2005
(51) Int. Cl.: C08G 18/02, C08G 18/76, C08G 18/79, C07C 267/00, B32B 7/12

(54) **VERFAHREN ZUR KASCHIERUNG UNTER VERWENDUNG SPEZIELLER VERNETZER MIT CARBODIIMID-GRUPPEN**
METHOD FOR LAMINATING USING SPECIAL CROSS-LINKED CARBODIIMIDE GROUPS
PROCEDE DE CONTRECOLLAGE AU MOYEN D'AGENTS RETICULANTS SPECIAUX COMPORTANT DES GROUPES CARBODIIMIDE

(30) Priorität: 23.12.2004 DE 102004063380
(43) Veröffentlichungstag der Anmeldung: 12.09.2007
(73) Patentinhaber: BASF SE, 67056 Ludwigshafen (DE)
(72) Erfinder: BURGHARDT, Andre, 67240 Bobenheim-Roxheim (DE); HÄBERLE, Karl, 67346 Speyer (DE); LICHT, Ulrike, 68309 Mannheim (DE); NÖRENBERG, Ralf, 67063 Ludwigshafen (DE); SCHUMACHER, Karl-Heinz, 67433 Neustadt (DE)
(86) Internationale Anmeldenummer: PCT/EP2005/013793
(87) Internationale Veröffentlichungsnummer: WO 2006/069703

(56) Entgegenhaltungen:
- EP-A- 0 686 626
- US-A- 5 958 516

## Beschreibung

Die Erfindung betrifft ein Verfahren zur Verklebung von Substraten, unter Verwendung eines Klebstoffs, der ein Polymer als Bindemittel und eine Carbodiimid-Gruppen enthaltende Verbindung I als Vernetzer enthält, dadurch gekennzeichnet, dass die Carbodiimid-Gruppen enthaltende Verbindung erhältlich ist durch Umsetzung von
a) Carbodiimiden des 1,3-Bis-(1 methyl-1-isocyanatoethyl) benzol, 1,4-Bis-(1 methyl-1-isocyanatoethyl) benzol oder deren Gemische mit
b) Polyalkylenoxiden mit mindestens zwei gegenüber Isocyanat reaktiven Gruppen, vorzugsweise mindestens zwei Hydroxylgruppen und
c) gegebenenfalls weiteren mit a) oder b) reaktiven Verbindungen.

Die Erfindung betrifft weiterhin spezielle Vernetzer mit Carbodiimidgruppen, welche sich für diese Verfahren besonders eignen.

In der Automobil- und Möbelindustrie werden häufig Bauteile mit einer Kunststofffolie kaschiert. Hierbei wird vom Hersteller des kaschierten Bauteils das Substrat und/oder die aufzukaschierende Folie mit einem Klebstoff beschichtet und, gegebenenfalls nach thermischer Aktivierung des Klebstoffes, die beiden Fügeteile miteinander verklebt, im Allgemeinen unter Aufwendung von Druck.

Als Klebstoff werden häufig wässrige Polymer-Dispersionen verwendet. Es ist auch allgemein gebräuchlich, der Dispersion einen Vernetzer zuzufügen, um eine ausreichende Wärmestandfestigkeit des Klebeverbunds zu gewährleisten. Als Vernetzer werden häufig Isocyanate verwendet, die jedoch wegen ihrer kurzen Lebensdauer, die im Allgemeinen einen Arbeitstag nicht überschreitet, der Klebstoffdispersion erst kurz vor der Anwendung zugesetzt werden können. Auch in der trockenen Klebstoffbeschichtung tritt nach kurzer Zeit eine Reaktion des Vernetzers ein, so dass keine lagerfähigen mit Klebstoff beschichteten Kaschierfolien hergestellt werden können.

Mit Klebstoff beschichtete, lagerfähige Kaschierfolien sind in der nicht vorveröffentlichten DE-A 103 30 748 (Aktenzeichen 10330748.6) beschrieben; der verwendete Klebstoff enthält Verbindungen mit Carbodiimidgruppen als Vernetzer.

Carbodiimidgruppen enthaltende Verbindungen, auch solche auf Basis von Bis-(1 methyl-1-isocyanatoethyl) benzol (kurz TMXDI), sind für unterschiedliche Verwendungen aus EP-A 686 626, DE-A 198 21 666 und DE-A 43 18 979 bekannt.

Die anwendungstechnischen Eigenschaften der bei der Kaschierung verwendeten Klebstoffe soll weiter verbessert werden. Neben einer guten Haftung auf den zu verklebenden Substraten ist eine hohe Festigkeit des erhaltenen Verbundes, insbesondere auch bei hohen Temperaturen (Wärmestandfestigkeit), erforderlich.

Aufgabe der vorliegenden Erfindung war daher ein Klebeverfahren, insbesondere Kaschierverfahren, welches die vorstehenden Anforderungen erfüllt und Produkte mit möglichst guten anwendungstechnischen Eigenschaften ermöglicht.

Aufgabe war es auch, Klebstoffe und Vernetzer, welche sich für ein derartiges Verfahren eignen, zu finden.

Demgemäß wurde das eingangs definierte Verfahren gefunden. Gefunden wurden auch Klebstoffe und Vernetzer, welche sich für dieses Verfahren eignen.

Der in dem erfindungsgemäßen Verfahren verwendete Klebstoff enthält eine Carbodiimidgruppen enthaltende Verbindung (kurz Verbindung 1) als Vernetzer.

Verbindung I ist aufgebaut aus den Carbodiimiden a), Polyalkylenoxiden b) und gegebenenfalls weitere reaktiven Verbindungen c).

### Zu a):

Bei 1,3 Bis-(1 methyl-1-isocyanatoethyl) benzol (auch m-Tetramethylxylyldüsocyanat = m-TMXDI) handelt es sich um ein Diisocyanat der folgenden Formel:

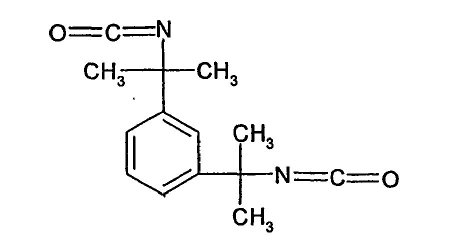

Bei 1,4 Bis-(1 methyl-1-isocyanatoethyl)benzol (auch p- Tetramethylxylyldiisocyanat = p-TMXDI) handelt es sich um ein Diisocyanat der folgenden Formel:

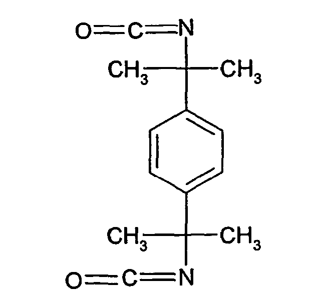

Bei a) kann es sich um Carbodiimide auf Basis von m-TMXDI, p-TMXDI oder deren Gemische handeln; bevorzugt sind Carbodiimide auf Basis von m-TMXDI.

Carbodiimidgruppen sind in einfacher Weise aus zwei Isocyanatgruppen unter Abspaltung von Kohlendioxid erhältlich:

-R-N=C=O + O=C=N-R

-R-N=C=N-R + CO₂

Ausgehend von TMXDI sind so Carbodiimide a) mit mehreren Carbodiimidgruppen und gegebenenfalls Isocyanatgruppen, insbesondere endständigen Isocyanatgruppen, erhältlich.

Bevorzugt sind Carbodiimide a) mit im Mittel 1 bis 20, vorzugsweise 1 bis 15 besonders bevorzugt 2 bis 10 Carbodiimidgruppen.

Das zahlenmittlere Molgewicht Mₙ der Carbodiimide a) beträgt vorzugsweise 100 bis 10000 besonders bevorzugt 200 bis 5000 und ganz besonders 500 bis 2000 g/mol.

Das zahlenmittlere Molgewicht wird bestimmt durch Endgruppenanalyse der Diisocyanate (d. h. Verbrauch der Isocyanatgruppen durch Carbodiimidbildung, s. unten) oder falls die Endgruppenanalyse nicht möglich ist, durch Gelpermeationschromatographie (Polystyrolstandard, THF als Elutionsmittel).

### Zu b):

Polyalkylenoxide b) haben mindestens 2, vorzugsweise 2 bis 4, besonders bevorzugt 2 gegenüber Isocyanat reaktive Gruppen.

Insbesondere handelt es sich bei den reaktiven Gruppen um Hydroxylgruppen.

Vorzugsweise handelt es sich daher bei b) um ein Diol.

Bei den Alkylenoxidgruppen kann es sich insbesondere um Ethylenoxidgruppen (- CH₂- CH₂ - O - ) oder Propylenoxidgruppen 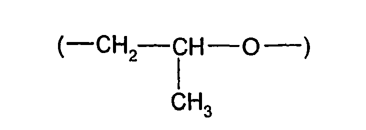 oder deren Gemische handeln. Bevorzugt sind Ethylenoxidgruppen.
b) besteht demnach bevorzugt ausschließlich aus Alkylenoxidgruppen und den reaktiven Gruppen, insbesondere Hydroxylgruppen; letztere sind insbesonders endständig angeordnet.

Besonders bevorzugte Verbindungen b) sind solche der Formel

H-O-(CH₂-CHR₁-O-)ₙH

Worin R¹ für eine CH₃ Gruppe oder vorzugsweise für ein H-Atom steht und n für eine ganze Zahl von 1 bis 100, vorzugsweise 1 bis 50.

### Zu c):

Verbindungen l können auch noch weitere Verbindungen c) als Aufbaukomponenten enthalten.

In Betracht kommen z. B. sonstige Isocyanate oder Verbindungen, die mit Isocyanatgruppen reaktive Gruppen, wie Hydroxyl- oder Aminogruppen tragen.
Weitere Verbindungen c) sind im Rahmen der vorliegenden Erfindung jedoch nicht notwendig und werden daher vorzugsweise nur in untergeordneten Mengen von weniger als 5 Gew. %, bezogen auf die Summe von a) + b) + c), besonders bevorzugt gar nicht mit verwendet.

Verbindungen I sind in einfacher Weise durch Umsetzung der Isocyanatgruppen (i) mit der gegenüber Isocyanat reaktiven Gruppen (ii) erhältlich. Bei der Umsetzung beträgt das Verhältnis (i) : (ii) vorzugsweise 1 : 10 bis 10 : 1.

Verbindungen 1 sind vorzugsweise bei Raumtemperatur (25°C, 1 bar) flüssig.

Verbindungen I bestehen vorzugsweise zu mindestens 40 bis 80 Gew. %, aus Alkylenoxidgruppen, besonders bevorzugt zu 40 bis 70 Gew. % aus Alkylenoxidgruppen, insbesondere Ethylenoxidgruppen.

Verbindungen I haben im Mittel vorzugsweise 2 bis 3, besonders bevorzugt 2 Hydroxylgruppen pro Molekül, der Gehalt an Isocyanatgruppen und sonstigen gegenüber Isocyanat reaktiven Gruppen ist vorzugsweise im Mittel kleiner 0,1 pro Molekül.

Besonders bevorzugt enthalten Verbindungen I keine Isocyanatgruppen oder sonstige gegenüber Isocyanat reaktiven Gruppen.

Auch ionische Gruppen oder in ionischen Gruppen überführbare Gruppen sind vorzugsweise nicht in Verbindung l enthalten.

Der im erfindungsgemäßen Verfahren verwendende Klebstoff enthält mindestens ein polymeres Bindemittel und gegebenenfalls Zusatzstoffe wie Füllstoffe, Verdicker, Entschäumer, Farbstoffe, Pigmente etc.

Bei dem polymeren Bindemittel handelt es sich vorzugsweise um ein Polyurethan, ein radikalisch polymerisiertes Polymer oder deren Gemische. Die polymeren Bindemittel liegen vorzugsweise in Form von wässrigen Dispersionen vor.

Die Polyurethane bestehen vorzugsweise überwiegend aus Polyisocyanaten, insbesondere Diisocyanaten und Polyesterdiolen, Polyetherdiolen oder deren Gemische.

Vorzugsweise ist das Polyurethan zu mindestens 40 Gew. %, besonders bevorzugt zu mindestens 60 Gew. % und ganz besonders bevorzugt zu mindestens 80 Gew. % aus Diisocyanaten, Polyetherdiolen und/oder Polyesterdiolen aufgebaut.

Vorzugsweise hat das Polyurethan einen Erweichungspunkt oder Schmelzpunkt im Bereich von -50 bis 150°C, besonders bevorzugt von 0 bis 100, und ganz besonders bevorzugt von 10 bis 90°C.

Besonders bevorzugt hat das Polyurethan einen Schmelzpunkt im vorstehenden Temperaturbereich.

Bevorzugt enthält das Polyurethan dazu Polyesterdiole in einer Menge von mehr als 10 Gew. %, bezogen auf das Polyurethan.

Insgesamt ist das Polyurethan vorzugsweise aufgebaut aus:
a) Diisocyanaten,
b) Diolen, von denen
   b₁₎ 10 bis 100 mol%, bezogen auf die Gesamtmenge der Diole (b), ein Molekulargewicht von 500 bis 5000 g/mol aufweisen,
   b₂₎ 0 bis 90 mol-%, bezogen auf die Gesamtmenge der Diole (b), ein Molekulargewicht von 60 bis 500 g/mol aufweisen,
c) von den Monomeren (a) und (b) verschiedene Monomere mit wenigstens einer Isocyanatgruppe oder wenigstens einer gegenüber Isocyanatgruppen reaktiven Gruppe, die darüber hinaus wenigstens eine hydrophile Gruppe oder eine potentiell hydrophile Gruppe tragen, wodurch die Wasserdispergierbarkeit der Polyurethane bewirkt wird,
d) gegebenenfalls weiteren von den Monomeren (a) bis (c) verschiedenen mehrwertigen Verbindungen mit reaktiven Gruppen, bei denen es sich um alkoholische Hydroxylgruppen, primäre oder sekundäre Aminogruppen oder Isocyanatgruppen handelt und
e) gegebenenfalls von den Monomeren (a) bis (d) verschiedenen einwertigen Verbindungen mit einer reaktiven Gruppe, bei der es sich um eine alkoholische Hydroxylgruppe, eine primäre oder sekundäre Aminogruppe oder eine Isocyanatgruppe handelt.

Insbesondere zu nennen sind als Monomere (a) Diisocyanate X(NCO)₂, wobei X für einen aliphatischen Kohlenwasserstoffrest mit 4 bis 15 Kohlenstoffatomen, einen cycloaliphatischen oder aromatischen Kohlenwasserstoffrest mit 6 bis 15 Kohlenstoffatomen oder einen araliphatischen Kohlenwasserstoffrest mit 7 bis 15 Kohlenstoffatomen steht. Beispiele derartiger Diisocyanate sind Tetramethylendiisocyanat, Hexamethylendiisocyanat, Dodecamethylendiisocyanat, 1,4-Diisocyanatocyclohexan, 1-Isocyanato-3,5,5-trimethyl-5-isocyanatomethylcyclohexan (IPDI), 2,2-Bis-(4-isocyanatocyclohexyl)-propan, Trimethylhexandiisocyanat, 1,4-Diisocyanatobenzol, 2,4-Diisocyanatotoluol, 2,6-Diisocyanatotoluol, 4,4'-Diisocyanato-diphenylmethan, 2,4'-Diisocyanato-diphenylmethan, p-Xylylendüsocyanat, Tetramethylxylylendüsocyanat (TMXDI), die Isomeren des Bis-(4-isocyanatocyclohexyl)methans (HMDI) wie das trans/trans-, das cis/cis- und das cis/trans-Isomere sowie aus diesen Verbindungen bestehende Gemische.

Derartige Diisocyanate sind im Handel erhältlich.

Als Gemische dieser Isocyanate sind besonders die Mischungen der jeweiligen Strukturisomeren von Diisocyanatotoluol und Diisocyanato-diphenylmethan von Bedeutung, insbesondere ist die Mischung aus 80 mol% 2,4-Diisocyanatotoluol und 20 mol-% 2,6-Diisocyanatotoluol geeignet. Weiterhin sind die Mischungen von aromatischen Isocyanaten wie 2,4-Diisocyanatotoluol und/oder 2,6-Diisocyanatotoluol mit aliphatischen oder cycloaliphatischen Isocyanaten wie Hexamethylendiisocyanat oder IPDI besonders vorteilhaft, wobei das bevorzugte Mischungsverhältnis der aliphatischen zu aromatischen Isocyanate 4 : 1 bis 1 : 4 beträgt.

Zum Aufbau der Polyurethane kann man als Verbindungen außer den vorgenannten auch Isocyanate einsetzen, die neben den freien Isocyanatgruppen weitere verkappte Isocyanatgruppen, z. B. Uretdiongruppen tragen.

Im Hinblick auf gute Filmbildung und Elastizität kommen als Diole (b) vornehmlich höhermolekulare Diole (b1) in Betracht, die ein Molekulargewicht von etwa 500 bis 5000, vorzugsweise von etwa 1000 bis 3000 g/mol haben. Es handelt sich hierbei um das zahlenmittlere Molgewicht Mn. Mn ergibt sich durch Bestimmung der Anzahl der Endgruppen (OH-Zahl).

Bei den Diolen (b1) kann es sich um Polyesterpolyole handeln, die z. B. aus Ullmanns Encyklopädie der technischen Chemie, 4. Auflage, Band 19, S. 62 bis 65 bekannt sind. Bevorzugt werden Polyesterpolyole eingesetzt, die durch Umsetzung von zweiwertigen Alkoholen mit zweiwertigen Carbonsäuren erhalten werden. Anstelle der freien Polycarbonsäuren können auch die entsprechenden Polycarbonsäureanhydride oder entsprechende Polycarbonsäureester von niederen Alkoholen oder deren Gemische zur Herstellung der Polyesterpolyole verwendet werden. Die Polycarbonsäuren können aliphatisch, cycloaliphatisch, araliphatisch, aromatisch oder heterocyclisch sein und gegebenenfalls, z. B. durch Halogenatome, substituiert und/oder ungesättigt sein. Als Beispiele hierfür seien genannt: Korksäure, Azelainsäure, Phthalsäure, Isophthalsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimere Fettsäuren. Bevorzugt sind Dicarbonsäuren der allgemeinen Formel HOOC- (CH₂)_{y}-COOH, wobei y eine Zahl von 1 bis 20, bevorzugt eine gerade Zahl von 2 bis 20 ist, z. B. Bernsteinsäure, Adipinsäure, Sebacinsäure und Dodecandicarbonsäure.

Als mehrwertige Alkohole kommen z. B. Ethylenglykol, Propan-1,2- diol, Propan-1,3-diol, Butan-1,3-diol, Buten-1,4-diol, Butin-1,4-diol, Pentan-1,5-diol, Neopentylglykol, Bis-(hydroxymethyl)-cyclohexane wie 1,4-Bis-(hydroxymethyl)cyclohexan, 2-Methyl-propan-1,3-diol, Methylpentandiole, ferner Diethylenglykol, Triethylenglykol, Tetraethylenglykol, Polyethylenglykol, Dipropylenglykol, Polypropylenglykol, Dibutylenglykol und Polybutylenglykole in Betracht. Bevorzugt sind Alkohole der allgemeinen Formel HO-(CH₂)ₓ-OH, wobei x eine Zahl von 1 bis 20, bevorzugt eine gerade Zahl von 2 bis 20 ist. Beispiele hierfür sind Ethylenglycol, Butan-1,4-diol, Hexan-1,6-diol, Octan-1,8-diol und Dodecan-1,12-diol. Weiterhin bevorzugt ist Neopentylglykol.

Ferner kommen gegebenenfalls auch Polycarbonat-Diole, wie sie z. B. durch Umsetzung von Phosgen mit einem Überschuss von den als Aufbaukomponenten für die Polyesterpolyole genannten niedermolekularen Alkohole erhalten werden können, in Betracht.

Gegebenenfalls können auch Polyesterdiole auf Lacton-Basis mitverwendet werden, wobei es sich um Homo- oder Mischpolymerisate von Lactonen, bevorzugt um endständige Hydroxylgruppen aufweisende Anlagerungsprodukte von Lactonen an geeignete difunktionelle Startermoleküle handelt. Als Lactone kommen bevorzugt solche in Betracht, die sich von Verbindungen der allgemeinen Formel HO-(CH₂)_{z}-COOH ableiten, wobei z eine Zahl von 1 bis 20 ist und ein H-Atom einer Methyleneinheit auch durch einen C₁- bis C₄-Alkylrest substituiert sein kann. Beispiele sind ε-Caprolacton, ß-Propiolacton, γ-Butyrolacton und/oder Methyl-γ-caprolacton sowie deren Gemische. Geeignete Starterkomponenten sind z. B. die vorstehend als Aufbaukomponente für die Polyesterpolyole genannten niedermolekularen zweiwertigen Alkohole. Die entsprechenden Polymerisate des ε-Caprolactons sind besonders bevorzugt. Auch niedere Polyesterdiole oder Polyetherdiole können als Starter zur Herstellung der Lacton-Polymerisate eingesetzt sein. Anstelle der Polymerisate von Lactonen können auch die entsprechenden, chemisch äquivalenten Polykondensate der den Lactonen entsprechenden Hydroxycarbonsäuren, eingesetzt werden.

Polyetherdiole sind insbesondere durch Polymerisation von Ethylenoxid, Propylenoxid, Butylenoxid, Tetrahydrofuran, Styroloxid oder Epichlorhydrin mit sich selbst, z. B. in Gegenwart von BF₃ oder durch Anlagerung dieser Verbindungen gegebenenfalls im Gemisch oder nacheinander, an Startkomponenten mit reaktionsfähigen Wasserstoffatomen, wie Alkohole oder Amine, z. B. Wasser, Ethylenglykol, Propan-1,2-diol, Propan-1,3-diol, 2,2-Bis(4-hydroxyphenyl)-propan oder Anilin erhältlich. Besonders bevorzugt sind Polypropylenoxid, Polytetrahydrofuran eines Molekulargewichts von 240 bis 5000, und vor allem 500 bis 4500.

Unter b₁₎ fallen nur Polyetherdiole, die zu weniger als 20 Gew. % aus Ethylenoxid bestehen. Polyetherdiole mit mindestens 20 Gew. % sind hydrophile Polyetherdiole, welche zu den Monomeren c) zählen.

Gegebenenfalls können auch Polyhydroxyolefine mitverwendet werden, bevorzugt solche mit 2 endständigen Hydroxylgruppen, z. B. α,-ω-Dihydroxypolybutadien, α,-ω-Dihydroxypolymethacrylester oder α,-ω-Dihydroxypolyacrylester als Monomere (c1).

Solche Verbindungen sind beispielsweise aus der EP-A 0622378 bekannt. Weitere geeignete Polyole sind Polyacetale, Polysiloxane und Alkydharze.

Bevorzugt handelt es sich bei mindestens 95 mol-% der Diole b₁₎ um Polyesterdiole. Besonders bevorzugt werden als Diole b₁₎ ausschließlich Polyesterdiole verwendet.

Die Härte und der Elastizitätsmodul der Polyurethane lassen sich erhöhen, wenn als Diole (b) neben den Diolen (b1) noch niedermolekulare Diole (b2) mit einem Molekulargewicht von etwa 60 bis 500, vorzugsweise von 62 bis 200 g/mol, eingesetzt werden.

Als Monomere (b2) werden vor allem die Aufbaukomponenten der für die Herstellung von Polyesterpolyolen genannten kurzkettigen Alkandiole eingesetzt, wobei die unverzweigten Diole mit 2 bis 12 C-Atomen und einer gradzahligen Anzahl von C-Atomen sowie Pentan-1,5-diol und Neopentylglykol bevorzugt werden.

Als Diole b₂₎ kommen z. B. Ethylenglykol, Propan-1,2-diol, Propan-1,3-diol, Butan-1,3-diol, Buten-1,4-diol, Butin-1,4-diol, Pentan-1,5-diol, Neopentylglykol, Bis-(hydroxymethyl)-cyclohexane wie 1,4-Bis-(hydroxymethyl)cyclohexan, 2-Methyl-propan-1,3-diol, Methylpentandiole, ferner Diethylenglykol, Triethylenglykol, Tetraethylenglykol, Polyethylenglykol, Dipropylenglykol, Polypropylenglykol, Dibutylenglykol und Polybutylenglykole in Betracht. Bevorzugt sind Alkohole der allgemeinen Formel HO-(CH₂)ₓ-OH, wobei x eine Zahl von 1 bis 20, bevorzugt eine gerade Zahl von 2 bis 20 ist. Beispiele hierfür sind Ethylenglycol, Butan-1,4-diol, Hexan-1,6-diol, Octan-1,8-diol und Dodecan-1,12-diol. Weiterhin bevorzugt ist Neopentylglykol.

Bevorzugt beträgt der Anteil der Diole (b₁), bezogen auf die Gesamtmenge der Diole (b) 10 bis 100 mol% und der Anteil der Monomere (b₂), bezogen auf die Gesamtmenge der Diole (b) 0 bis 90 mol-%. Besonders bevorzugt beträgt das Verhältnis der Diole (b1) zu den Monomeren (b2) 0,1 : 1 bis 5:1, besonders bevorzugt 0,2 : 1 bis 2 : 1.

Um die Wasserdispergierbarkeit der Polyurethane zu erreichen, enthalten die Polyurethane von den Komponenten (a), (b) und (d) verschiedene Monomere (c), die wenigstens eine Isocyanatgruppe oder wenigstens eine gegenüber Isocyanatgruppen reaktive Gruppe und darüberhinaus wenigstens eine hydrophile Gruppe oder eine Gruppe, die sich in eine hydrophile Gruppe überführen lässt, tragen, als Aufbaukomponente. Im folgenden Text wird der Begriff "hydrophile Gruppen oder potentiell hydrophile Gruppen" mit "(potentiell) hydrophile Gruppen" abgekürzt. Die (potentiell) hydrophilen Gruppen reagieren mit Isocyanaten wesentlich langsamer als die funktionellen Gruppen der Monomere, die zum Aufbau der Polymerhauptkette dienen.

Der Anteil der Komponenten mit (potentiell) hydrophilen Gruppen an der Gesamtmenge der Komponenten (a), (b), (c), (d) und (e) wird im allgemeinen so bemessen, dass die Molmenge der (potentiell) hydrophilen Gruppen, bezogen auf die Gewichtsmenge aller Monomere (a) bis (e), 30 bis 1000, bevorzugt 50 bis 500 und besonders bevorzugt 80 bis 300 mmol/kg beträgt.

Bei den (potentiell) hydrophilen Gruppen kann es sich um nichtionische oder bevorzugt um (potentiell) ionische hydrophile Gruppen handeln.

Als nichtionische hydrophile Gruppen kommen insbesondere Polyethylenglycolether aus vorzugsweise 5 bis 100, bevorzugt 10 bis 80 Ethylenoxid-Wiederholungseinheiten, in Betracht. Der Gehalt an Polyethylenoxid-Einheiten beträgt im allgemeinen 0 bis 10, bevorzugt 0 bis 6 Gew. %, bezogen auf die Gewichtsmenge aller Monomere (a) bis (e).

Bevorzugte Monomere mit nichtionischen hydrophilen Gruppen sind Polyethylenoxiddiole mit mindestens 20 Gew. % Ethylenoxid, Polyethylenoxidmonoole sowie die Reaktionsprodukte aus einem Polyethylenglykol und einem Diisocyanat, die einen endständig veretherten Polyethylenglykolrest tragen. Derartige Diisocyanate sowie Verfahren zu deren Herstellung sind in den Patentschriften US-A 3 905 929 und US-A 3 920 598 angegeben.

Ionische hydrophile Gruppen sind vor allem anionische Gruppen wie die Sulfonat-, die Carboxylat- und die Phosphatgruppe in Form ihrer Alkalimetall- oder Ammoniumsalze sowie kationische Gruppen wie Ammonium-Gruppen, insbesondere protonierte tertiäre Aminogruppen oder quartäre Ammoniumgruppen.

Potentiell ionische hydrophile Gruppen sind vor allem solche, die sich durch einfache Neutralisations-, Hydrolyse- oder Quaternisierungsreaktionen in die oben genannten ionischen hydrophilen Gruppen überführen lassen, also z. B. Carbonsäuregruppen oder tertiäre Aminogruppen.

(Potentiell) ionische Monomere (c) sind z. B. in Ullmanns Encyklopädie der technischen Chemie, 4. Auflage, Band 19, S. 311-313 und beispielsweise in der DE-A 1 495 745 ausführlich beschrieben.

Als (potentiell) kationische Monomere (c) sind vor allem Monomere mit tertiären Aminogruppen von besonderer praktischer Bedeutung, beispielsweise: Tris-(hydroxyalkyl)-amine, N,N'-Bis(hydroxyalkyl)-alkylamine, N-Hydroxyalkyl-dialkylamine, Tris-(aminoalkyl)-amine, N,N'-Bis(aminoalkyl)-alkylamine, N-Aminoalkyl-dialkylamine, wobei die Alkylreste und Alkandiyl-Einheiten dieser tertiären Amine unabhängig voneinander aus 1 bis 6 Kohlenstoffatomen bestehen. Weiterhin kommen tertiäre Stickstoffatome aufweisende Polyether mit vorzugsweise zwei endständigen Hydroxylgruppen, wie sie z. B. durch Alkoxylierung von zwei an Aminstickstoff gebundene Wasserstoffatome aufweisende Amine, z. B. Methylamin, Anilin oder N,N'-Dimethylhydrazin, in an sich üblicher Weise zugänglich sind, in Betracht. Derartige Polyether weisen im allgemeinen ein zwischen 500 und 6000 g/mol liegendes Molgewicht auf.

Diese tertiären Amine werden entweder mit Säuren, bevorzugt starken Mineralsäuren wie Phosphorsäure, Schwefelsäure, Halogenwasserstoffsäuren oder starken organischen Säuren oder durch Umsetzung mit geeigneten Quaternisierungsmitteln wie C₁-bis C₆-Alkylhalogeniden oder Benzylhalogeniden, z. B. Bromiden oder Chloriden, in die Ammoniumsalze überführt.

Als Monomere mit (potentiell) anionischen Gruppen kommen üblicherweise aliphatische, cycloaliphatische, araliphatische oder aromatische Carbonsäuren und Sulfonsäuren in Betracht, die mindestens eine alkoholische Hydroxylgruppe oder mindestens eine primäre oder sekundäre Aminogruppe tragen. Bevorzugt sind Dihydroxyalkylcarbonsäuren, vor allem mit 3 bis 10 Kohlenstoffatomen, wie sie auch in der US-A 3 412 054 beschrieben sind. Insbesondere sind Verbindungen der allgemeinen Formel (c₁) 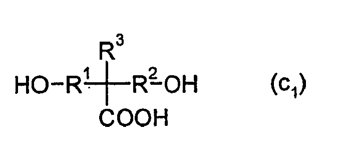 in welcher R¹ und R² für eine C₁- bis C₄-Alkandiyl-(Einheit) und R³ für eine C₁- bis C₄-Alkyl-(Einheit) steht und vor allem Dimethylolpropionsäure (DMPA) bevorzugt.

Weiterhin eignen sich entsprechende Dihydroxysulfonsäuren und Dihydroxyphosphonsäuren wie 2,3-Dihydroxypropanphosphonsäure.

Ansonsten geeignet sind Dihydroxylverbindungen mit einem Molekulargewicht über 500 bis 10000 g/mol mit mindestens 2 Carboxylatgruppen, die aus der DE-A 3 911 827 bekannt sind. Sie sind durch Umsetzung von Dihydroxylverbindungen mit Tetracarbonsäuredianhydriden wie Pyromellitsäuredianhydrid oder Cyclopentantetracarbonsäuredianhydrid im Molverhältnis 2 : 1 bis 1,05: 1 in einer Polyadditionsreaktion erhältlich. Als Dihydroxylverbindungen sind insbesondere die als Kettenverlängerer aufgeführten Monomere (b2) sowie die Diole (b1) geeignet.

Als Monomere (c) mit gegenüber Isocyanaten reaktiven Aminogruppen kommen Aminocarbonsäuren wie Lysin, β-Alanin oder die in der DE-A 2 034 479 genannten Addukte von aliphatischen diprimären Diaminen an a,β-ungesättigte Carbon- oder Sulfonsäuren in Betracht.

Solche Verbindungen gehorchen beispielsweise der Formel (c₂)

H₂N-R⁴-NH-R⁵-X (c₂)

in der
- R⁴ und R⁵ unabhängig voneinander für eine C₁- bis C₆-Alkandiyl-Einheit, bevorzugt Ethylen
und X für COOH oder SO₃H stehen.

Besonders bevorzugte Verbindungen der Formel (C₂) sind die N-(2-Aminoethyl)-2-aminoethancarbonsäure sowie die N-(2-Aminoethyl)-2-aminoethansulfonsäure bzw. die entsprechenden Alkalisalze, wobei Na als Gegenion besonders bevorzugt ist.

Weiterhin besonders bevorzugt sind die Addukte der oben genannten aliphatischen diprimären Diamine an 2-Acrylamido-2-methylpropansulfonsäure, wie sie z. B. in der DE-B 1 954 090 beschrieben sind.

Sofern Monomere mit potentiell ionischen Gruppen eingesetzt werden, kann deren Überführung in die ionische Form vor, während, jedoch vorzugsweise nach der Isocyanat-Polyaddition erfolgen, da sich die ionischen Monomeren in der Reaktionsmischung häufig nur schwer lösen. Besonders bevorzugt liegen die Sulfonat- oder Carboxylatgruppen in Form ihrer Salze mit einem Alkaliion oder einem Ammoniumion als Gegenion vor.

Die Monomere (d), die von den Monomeren (a) bis (c) verschieden sind und welche gegebenenfalls auch Bestandteile des Polyurethans sind, dienen im allgemeinen der Vernetzung oder der Kettenverlängerung. Es sind im allgemeinen mehr als zweiwertige nicht-phenolische Alkohole, Amine mit 2 oder mehr primären und/oder sekundären Aminogruppen sowie Verbindungen, die neben einer oder mehreren alkoholischen Hydroxylgruppen eine oder mehrere primäre und/oder sekundäre Aminogruppen tragen.

Alkohole mit einer höheren Wertigkeit als 2, die zur Einstellung eines gewissen Verzweigungs- oder Vernetzungsgrades dienen können, sind z. B. Trimethylolpropan, Glycerin oder Zucker.

Ferner kommen Monoalkohole in Betracht, die neben der Hydroxyl-Gruppe eine weitere gegenüber Isocyanaten reaktive Gruppe tragen wie Monoalkohole mit einer oder mehreren primären und/oder sekundären Aminogruppen, z. B. Monoethanolamin.

Polyamine mit 2 oder mehr primären und/oder sekundären Aminogruppen werden vor allem dann eingesetzt, wenn die Kettenverlängerung bzw. Vernetzung in-Gegenwart von Wasser stattfinden soll, da Amine in der Regel schneller als Alkohole oder Wasser mit Isocyanaten reagieren. Das ist häufig dann erforderlich, wenn wässerige Dispersionen von vernetzten Polyurethanen oder Polyurethanen mit hohem Molgewicht gewünscht werden. In solchen Fällen geht man so vor, dass man Prepolymere mit Isocyanatgruppen herstellt, diese rasch in Wasser dispergiert und anschließend durch Zugabe von Verbindungen mit mehreren gegenüber Isocyanaten reaktiven Aminogruppen kettenverlängert oder vernetzt.

Hierzu geeignete Amine sind im allgemeinen polyfunktionelle Amine des Molgewichtsbereiches von 32 bis 500 g/mol, vorzugsweise von 60 bis 300 g/mol, welche mindestens zwei Aminogruppen, ausgewählt aus der Gruppe der primären und sekundären Aminogruppen, enthalten. Beispiele hierfür sind Diamine wie Diaminoethan, Diaminopropane, Diaminobutane, Diaminohexane, Piperazin, 2,5-Dimethylpiperazin, Amino-3-aminomethyl-3,5,5-trimethyl-cyclohexan (Isophorondiamin, IPDA), 4,4'-Diaminodicyclohexylmethan, 1,4-Diaminocyclohexan, Aminoethylethanolamin, Hydrazin, Hydrazinhydrat oder Triamine wie Diethylentriamin oder 1,8-Diamino-4-aminomethyloctan.

Die Amine können auch in blockierter Form, z. B. in Form der entsprechenden Ketimine (siehe z. B. CA-A 1 129 128), Ketazine (vgl. z. B. die US-A 4 269 748) oder Aminsalze (siehe US-A 4 292 226) eingesetzt werden. Auch Oxazolidine, wie sie beispielsweise in der US-A 4 192 937 verwendet werden, stellen verkappte Polyamine dar, die für die Herstellung der erfindungsgemäßen Polyurethane zur Kettenverlängerung der Prepolymeren eingesetzt werden können. Bei der Verwendung derartiger verkappter Polyamine werden diese im allgemeinen mit den Prepolymeren in Abwesenheit von Wasser vermischt und diese Mischung anschließend mit dem Dispersionswasser oder einem Teil des Dispersionswassers vermischt, so dass hydrolytisch die entsprechenden Polyamine freigesetzt werden.

Bevorzugt werden Gemische von Di- und Triaminen verwendet, besonders bevorzugt Gemische von Isophorondiamin (IPDA) und Diethylentriamin (DETA).

Die Polyurethane enthalten bevorzugt 1 bis 30, besonders bevorzugt 4 bis 25 mol-%, bezogen auf die Gesamtmenge der Komponenten (b) und (d) eines Polyamins mit mindestens 2 gegenüber Isocyanaten reaktiven Aminogruppen als Monomere (d).

Für den gleichen Zweck können auch als Monomere (d) höher als zweiwertige Isocyanate eingesetzt werden. Handelsübliche Verbindungen sind beispielsweise das Isocyanurat oder das Biuret des Hexamethylendiisocyanats.

Monomere (e), die gegebenenfalls mitverwendet werden, sind Monoisocyanate, Monoalkohole und monoprimäre und -sekundäre Amine. Im allgemeinen beträgt ihr Anteil maximal 10 mol-%, bezogen auf die gesamte Molmenge der Monomere. Diese monofunktionellen Verbindungen tragen üblicherweise weitere funktionelle Gruppen wie olefinische Gruppen oder Carbonylgruppen und dienen zur Einführung von funktionellen Gruppen in das Polyurethan, die die Dispergierung bzw. die Vernetzung oder weitere polymeranaloge Umsetzung des Polyurethans ermöglichen. In Betracht kommen hierfür Monomere wie Isopropenyl-a,a-dimethylbenzylisocyanat (TMI) und Ester von Acryl- oder Methacrylsäure wie Hydroxyethylacrylat oder Hydroxyethylmethacrylat.

Überzüge mit einem besonders guten Eigenschaftsprofil erhält man vor allem dann, wenn als Monomere (a) im wesentlichen nur aliphatische Diisocyanate, cycloaliphatische Diisocyanate oder araliphatische Diisocyanate eingesetzt werden.

Diese Monomerkombination wird in hervorragender Weise ergänzt als Komponente (c) durch Diaminosulfonsäure-Alkali-Salze; ganz besonders durch die N-(2-Aminoethyl)-2-aminoethansulfonsäure bzw. ihre entsprechenden Alkalisalze, wobei das Na-Salz am besten geeignet ist, und eine Mischung von DETA und IPDA als Komponente (d).

Auf dem Gebiet der Polyurethanchemie ist allgemein bekannt, wie das Molekulargewicht der Polyurethane durch Wahl der Anteile der miteinander reaktiven Monomere sowie des arithmetischen Mittels der Zahl der reaktiven funktionellen Gruppen pro Molekül eingestellt werden kann.

Normalerweise werden die Komponenten (a) bis (e) sowie ihre jeweiligen Molmengen so gewählt, dass das Verhältnis A : B mit
- A: der Molmenge an Isocyanatgruppen und
- B: der Summe aus der Molmenge der Hydroxylgruppen und der Molmenge der funktionellen Gruppen, die mit Isocyanaten in einer Additionsreaktion reagieren können

0,5 : 1 bis 2 : 1, bevorzugt 0,8 : 1 bis 1,5, besonders bevorzugt 0,9 : 1 bis 1,2 : 1 beträgt. Ganz besonders bevorzugt liegt das Verhältnis A : B möglichst nahe an 1 : 1.

Die eingesetzten Monomere (a) bis (e) tragen im Mittel üblicherweise 1,5 bis 2,5, bevorzugt 1,9 bis 2,1, besonders bevorzugt 2,0 Isocyanatgruppen bzw. funktionelle Gruppen, die mit Isocyanaten in einer Additionsreaktion reagieren können.

Die Polyaddition der Komponenten (a) bis (e) zur Herstellung des Polyurethans erfolgt vorzugsweise bei Reaktionstemperaturen von bis zu 180°C, bevorzugt bis zu 150°C unter Normaldruck oder unter autogenem Druck.

Die Herstellung von Polyurethanen, bzw. von wässrigen Polyurethandispersionen ist dem Fachmann bekannt.

Soweit Carbodiimide an das Polyurethan angebunden werden sollen, können bei der Herstellung der Polyurethane Carbodiimide mit endständigen Isocyanatgruppen (siehe oben) als Diisocyanate a) in der gewünschten Menge eingesetzt werden.

Bei dem polymeren Bindemittel kann es sich auch um ein radikalisch polymerisiertes Polymer handeln, d. h. das Polymer ist erhältlich durch radikalische Polymerisation von ethylenisch ungesättigten Verbindungen (Monomere).

Das Polymer besteht vorzugsweise zu mindestens 40 Gew. %, bevorzugt zu mindestens 60 Gew. %, besonders bevorzugt zu mindestens 80 Gew. % aus sogenannten Hauptmonomeren.

Die Hauptmonomeren sind ausgewählt aus C₁-C₂₀-Alkyl(meth)acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden, Vinylethern von 1 bis 10 C-Atome enthaltenden Alkoholen, aliphatischen Kohlenwasserstoffen mit 2 bis 8 C-Atomen und ein oder zwei Doppelbindungen oder Mischungen dieser Monomeren.

Zu nennen sind z. B. (Meth)acrylsäurealkylester mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z. B. Vinyllaurat, -stearat, Vinylpropionat, Versaticsäurevinylester und Vinylacetat.

Als vinylaromatische Verbindungen kommen Vinyltoluol, a- und p-Methylstyrol, a-Butylstyrol, 4-n-Butylstyrol, 4-n-Decylstyrol und vorzugsweise Styrol in Betracht. Beispiele für Nitrile sind Acrylnitril und Methacrylnitril.

Die Vinylhalogenide sind mit Chlor, Fluor oder Brom substituierte ethylenisch ungesättigte Verbindungen, bevorzugt Vinylchlorid und Vinylidenchlorid.

Als Vinylether zu nennen sind z. B. Vinylmethylether oder Vinylisobutylether. Bevorzugt wird Vinylether von 1 bis 4 C-Atome enthaltenden Alkoholen.

Als Kohlenwasserstoffe mit 2 bis 8 C-Atomen und ein oder zwei olefinischen Doppelbindungen seien Ethylen, Propylen, Butadien, Isopren und Chloropren genannt.

Als Hauptmonomere bevorzugt sind die C₁- bis C₁₀-Alkylacrylate und -methacrylate, insbesondere C₁- bis C₈-Alkylacrylate und -methacrylate und Vinylaromaten, insbesondere Styrol und deren Mischungen.

Als Hauptmonomere ebenfalls bevorzugt sind Vinylester, aliphatische Kohlenwasserstoffe und deren Mischungen, z.B. Vinylacetat, Ethylen und deren Mischungen.

Ganz besonders bevorzugt sind Methylacrylat, Methylmethacrylat, Ethylacrylat, n-Butylacrylat, n-Hexylacrylat, Octylacrylat und 2-Etyhlhexylacrylat, Styrol sowie Mischungen dieser Monomere.

Neben den Hauptmonomeren kann das Polymer weitere Monomere enthalten, z. B. Monomere mit Carbonsäure-, Sulfonsäure- oder Phosphonsäuregruppen. Bevorzugt sind Carbonsäuregruppen. Genannt seien z. B. Acrylsäure, Methacrylsäure, Itaconsäure, Maleinsäure oder Fumarsäure.

Weitere Monomere sind z. B. auch Hydroxylgruppen enthaltende Monomere, insbesondere C₁-C₁₀-Hydroxyalkyl(meth)acrylate, (Meth)acrylamid.

Als weitere Monomere seien darüber hinaus Phenyloxyethylglykolmono-(meth-)acrylat, Glycidylacrylat, Glycidylmethacrylat, Amino- (meth)acrylate wie 2-Aminoethyl-(meth-) acrylat genannt.

Als weitere Monomere seien auch vernetzende Monomere genannt.

Besonders bevorzugt besteht das Polymer zu mindestens 40 Gew. %, insbesondere mindestens 60 Gew. % und ganz besonders bevorzugt zu mindestens 80 Gew. % aus C₁-C₂₀-, insbesondere C₁-C₁₀Alkyl(meth)acrylaten.

Die Herstellung der Polymere erfolgt in einer bevorzugten Ausführungsform durch Emulsionspolymerisation, es handelt sich daher um ein Emulsionspolymerisat.

Die Herstellung kann jedoch z. B. auch durch Lösungspolymerisation und anschlie-ßende Dispergierung in Wasser erfolgen.

### Zum Klebstoff:

Zusammenfassend handelt es sich bei dem Klebstoff vorzugsweise um einen wässrigen Klebstoff. Das polymere Bindemittel liegt dazu vorzugsweise in Form einer wässrigen Dispersion vor. Weitere Zusatzstoffe können in einfacher Weise zur wässrigen Dispersion des polymeren Bindemittels gegeben werden.

Insbesondere können Verbindungen 1 einfach in die wässrige Dispersion eingerührt werden.

Der Gehalt an Verbindungen l beträgt vorzugsweise 0,1 bis10 Gew.-Teile, besonders bevorzugt 0,2 bis 8 Gew.-Teile auf 100 Gew. % polymeres Bindemittel (fest, ohne Wasser).

Der nach dem erfindungsgemäßen Verfahren verwendete Klebstoff enthält vorzugsweise 0,0001 bis 0,1 Mol, vorzugsweise 0,0005 bis 0,1 Mol, besonders bevorzugt 0,001 bis 0,1 Mol Carbodiimidgruppen auf 100 g Klebstoff; Wasser oder sonstige organische Lösemittel mit einem Siedepunkt unter 150°C bei 1 bar werden bei der Gewichtsmenge des Klebstoffs nicht berücksichtigt. Insbesondere ist der Gehalt an Carbodiimidgruppen nicht höher als 0,05 Mol/100 g Klebstoff.

Insbesondere handelt es sich bei mindestens 80 Mol %, besonders bevorzugt bei mindestens 90 Mol %, ganz besonders bevorzugt bei 100 Mol % der im Klebstoff vorhandenen Carbodiimidgruppen um Carbodiimidgruppen der Verbindung 1.

Weitere Zusatzstoffe sind z. B. Verdicker, Verlaufshilfsmittel, Entschäumer und Pigmente.

### Zum Verfahren:

Beim erfindungsgemäßen Verfahren können beliebige Substrate miteinander verklebt werden.

Insbesondere kann das Verfahren zur Herstellung von Möbeln, Fahrzeugen, z.B. zur Verklebung von Fahrzeuginnenverkleidungen, oder zur Herstellung von Schuhen angewendet werden.

Es können mehrere nicht flexible Substrate miteinander verklebt werden, es können auch nicht-flexible Substrate, z.B. Platten oder sonstige Formteile miteinander verklebt werden.

Besonders bevorzugt ist das erfindungsgemäße Verfahren ein Kaschierverfahren, bei dem nicht-flexible Substrate mit einem flexiblen, flächigen Substrat verklebt (kaschiert) werden.

Unter einem flexiblen Substrat wird dabei ein flächiges Substrat verstanden, welches sich bei einer Substratfläche von 50x50 cm, parallel zur Erdoberfläche an einer Seite festgehalten, durch das Eigengewicht durchbiegt.

Vorzugsweise handelt es sich um ein Substrat, welches auf Trommeln mit einem Au-ßendurchmesser von 80 cm aufgewickelt werden kann.

Vorzugsweise handelt es sich bei dem flexiblen Substrat um flächige Substrate mit einer Dicke kleiner 10 mm, insbesondere kleiner 5 mm, besonders bevorzugt kleiner 0,5 mm, ganz besonders bevorzugt kleiner 0,3 mm.

Insbesondere kann es sich um Polymerfolien, Metallfolien, Vliese aus synthetischen oder natürlichen Fasern, beschichtetes oder unbeschichtetes Papier oder auch Furniere aus Holz oder Holzimitat handeln.

Besonders bevorzugt sind Polymerfolien, z. B. Folien aus Polyester, wie Polyethylenterephthalat, Polyolefinen wie Polyethylen, Polypropylen oder Polyvinylchlorid, aus Polyacetat, Polystyrol oder Copolymeren des Styrols.

Das flexible Substrat kann vorbehandelt sein, z. B. kann es mit Haftvermittlern beschichtet sein. Das flexible Substrat kann auch aus mehreren Schichten aufgebaut sein; in Betracht kommt z. B. eine Trägerschicht aus den vorstehenden Polymeren und auf diese Trägerschicht ein- oder beidseitig aufgebrachte Schutzbeschichtungen oder dekorative Beschichtungen, insbesondere kommt auch ein mehrschichtiges Substrat in Betracht, welches eine Schicht aus geschäumten Polymeren enthält.

Bei dem nicht-flexiblen Substrat kann es sich um einen Formkörper handeln, dessen äußere Form gleich bleibt, auch wenn dieser Formkörper mit seinem Eigengewicht belastet wird, z. B. indem der Formkörper freihängend nur an einer einzigen beliebigen Stelle gehalten wird.

Die vorstehenden Angaben beziehen sich auf Normalbedingungen (21 °C, 1 bar). Das nicht-flexible Substrat kann aus Holz oder Kunststoff, z. B. ABS (Acrylnitril-Butadien-Styrol) sein. Es kann sich z B. um Massivholz oder Sperrholz , Hartfaserplatten oder Mitteldichte Faserplatten (MDF Platten) handeln.

Insbesondere kann es sich Formteile handeln, welche aus synthetischen oder natürlichen Fasern oder Spänen aufgebaut sind. Die Formteile können eine beliebige Form haben.

Beim erfindungsgemäßen Verfahren kann zunächst das nicht-flexible Substrat mit dem Klebstoff beschichtet und gegebenenfalls nach Trocknung das flexible Substrat aufkaschiert werden.

Bei dem erfindungsgemäßen Verfahren kann aber insbesondere auch das flexible Substrat mit Klebstoff beschichtet werden. Die Beschichtung kann nach üblichen Auftragsverfahren erfolgen. Nach der Beschichtung erfolgt eine Trocknung, vorzugsweise bei Raumtemperatur oder Temperaturen bis zu 80°C, um Wasser oder sonstige Lösemittel zu entfernen, danach kann das beschichtete flexible Substrat aufkaschiert werden.

Die aufgebrachte Klebstoffmenge (auf dem flexiblen oder nicht-flexiblen Substrat) beträgt vorzugsweise 0,5 bis 100 g/m², besonders bevorzugt 2 bis 80 g/m², ganz besonders bevorzugt 10 bis 70 g/m².

Das mit Klebstoff beschichtete Substrat kann gelagert werden.

Das beschichtete flexible Substrat kann nach gegebenenfalls erfolgter Trocknung aufgewickelt werden. Vor der Weiterverarbeitung erfolgt im Allgemeinen eine Lagerung oder auch ein Transport, so dass bis zur Weiterverarbeitung eine Zeit von mehr als einer Woche, bzw. mehr als 3 Wochen, insbesondere auch mehr als 6 Wochen, bzw. mehr als 10 Wochen vergeht.

Das beschichtete Substrat ist lagerstabil, d. h. auch nach mehreren Wochen Lagerzeit kann das beschichtete Substrat mit unverändert guten Ergebnissen verarbeitet werden.

Zur Verklebung werden die zu verklebenden Teile zusammengefügt. Die Temperatur in der Klebstoffschicht beträgt vorzugsweise 20 bis 200°C, besonders bevorzugt 30 bis 180°C. Geeigneterweise kann dazu das beschichtete flexible Substrat auf entsprechende Temperaturen erwärmt werden.

Die Verklebung erfolgt vorzugsweise unter Druck, dazu können z. B. die zu verklebenden Teile mit einem Druck von 0,05 bis 50 N/mm² zusammengepresst werden.

Die erhaltenen Verbunde zeichnen sich durch hohe mechanische Festigkeit auch bei erhöhten Temperaturen (Wärmestandfestigkeit) oder unter sich stark ändernden Klimabedingungen (Klimabeständigkeit) aus. Diese guten Ergebnisse werden auch erzielt, wenn das beschichtete flexible Substrat vor der Verklebung lange Zeit, z. B. mehr als 3 Monate gelagert wurde.

Auch die Weichmacherbeständigkeit, Kratzfestigkeit und Blockfestigkeit sind gut. Der Carbodiimid-Vemetzer I ist in reiner Form, in Wasser und in der wässrigen Dispersion lagerstabil und lässt sich leicht in Wasser emulgieren.

Besondere Bedeutung hat das erfindungsgemäße Verfahren in der Auto- oder Möbelindustrie, z. B. bei der Verklebung von flexiblen Substraten auf Autoinnenteilen, wie Amaturenbretter, Türinnenverkleidungen und Hutablagen.

### Beispiele

### Synthese:

### Beispiel 1

250 g eines Carbodiimids auf Basis von TMXDI mit einem NCO-Gehalt von 6,7 Gew.-% wurden in einem Rührkolben vorgelegt. Dazu wurden 250 g eines Polyethylenoxid-Diols mit einem zahlenmittleren Molgewicht von 500 g gegeben und auf 120°C erwärmt. Nach drei Stunden Rühren war der NCO-Gehalt des Ansatzes auf < 0,1 Gew.-% gefallen.
Man erhielt ein gelbliches Öl, das durch Schütteln leicht in Wasser zu emulgieren war. Die Viskosität bei 50 °C betrug 2280 mPas.

### Vergleichsbeispiel 1

250 g eines Carbodiimids auf Basis von TMXDI mit einem NCO-Gehalt von 6,7 Gew. % wurden in einem Rührkolben vorgelegt. Dazu wurden 250 g eines Polyethylenoxidmonomethylether mit einem zahlenmittleren Molgewicht von 500 g gegeben und auf 120°C erwärmt. Nach drei Stunden Rühren war der NCO-Gehalt des Ansatzes auf < 0,1 Gew. % gefallen.

Man erhielt ein gelbliches Öl, das durch Schütteln leicht in Wasser zu emulgieren war. Die Viskosität bei 50 °C betrug 795 mPas.

### Synthese einer Polyurethan-Dispersion :

Es wurde nach Beispiel 3 der DE-A 2 804 609 eine Polyurethan-Dispersion hergestellt, wobei jedoch anstelle des Toluylendiisocyanates die äquimolare Menge Isophorondiisocanat verwendet wurde.

### Prüfung:

Es wurden in der Tabelle aufgeführten Klebstoff-Ansätze hergestellt:

### Mengenangaben in Gramm

| Klebstoff | 1 | 2 | 3 | 4 | 5 |
|---|---|---|---|---|---|
| | | | | | |
| Polyurethan-Dispersion | 100 | 100 | 100 | 100 | 100 |
| Collacral® LR8989 10%ig in Wasser | 6 | 6 | 6 | 6 | 6 |
| Carbodiimid (Beispiel 1) | 2 | 5 | | | |
| Carbodiimid (Vergleichsbeispiel 1) | | | 2 | 5 | |

Collacral® LR8989 ist ein Polyurethan-Assoziatiwerdicker der BASF AG.
Die Klebstoff-Ansätze wurden nach verschiedenen Lagerbedingungen, einer Wärmestandfestigkeitsprüfung unterzogen.

Hierzu wurden die Klebstoff-Ansätze mit Hilfe eines 2 mm Drahtrakels auf eine Hartfaserplatte der Größe 200x200 mm aufgezogen und in 5 min. bei 60°C getrocknet. Dann wurde die Klebstoffschicht mittels einer beheizbaren Presse auf 80°C erwärmt und eine PVC-Folie der Fa. Benecke der Größe 200x200 mm mit einem Druck von 0,1 N/mm² 30 Sekunden lang so aufkaschiert, dass die klebende Fläche 200x170 mm groß war.

Nach dem Kleberauftrag, der Trocknung und der Pressung wurden die Probekörper 24 Stunden bei Raumtemperatur konditioniert und anschließend die PVC-Folie in 30 mm breite Streifen mit 10 mm Zwischenraum geschnitten. Dabei wurde lediglich die Folie durchtrennt, die Hartfaserplatte blieb unbeschädigt. Die 10 mm breiten Zwischenstreifen wurden entfernt, die Enden der verbliebenen Prüfstreifen an einer Seite im Winkel von 180° umgeklappt und die Platte derart senkrecht in einen Umlufttrockenschrank montiert, dass sich die umgeklappten Enden der Prüfstreifen an der Oberseite befanden. Der Trockenschrank wurde auf 40°C aufgeheizt.
An die abgezogenen Enden der Prüfstreifen wurde ein 300 g-Gewicht angeklemmt und die Kante der Verklebung als Startpunkt der Prüfung markiert.
Die Prüfung beginnt bei 40°C. Nach jeweils 30 Minuten wird die Temperatur um 10°C erhöht. In der Aufheizphase sind die Prüfstreifen nicht belastet.

Beurteilt wird die Temperatur, gemessen in °C, bei der die Verklebung aufgegangen ist. Die Prüfung endet bei mehr als 130 Millimeter Ablösung der Prüfstreifen.

Prüfung 1: Wärmestandfestigkeit (WSF) nach 24 h Lagerung des Klebeverbunds bei RT

Ablaufstrecke in mm bei der jeweiligen Prüftemperatur, Mittelwerte aus fünf Messungen

| Temperatur (°C) | Beispiel 1 | Beispiel 2 | Beispiel 3 (Vergleich) | Beispiel 4 (Vergleich) | Beispiel 5 (Vergleich) |
|---|---|---|---|---|---|
| | | | | | |
| 40 | - | - | - | - | - |
| 50 | 1 | 2 | 1 | 2 | 18 |
| 60 | 1 | 1 | 1 | 2 | 118 |
| 70 | 1 | 1 | 1 | 1 | alle > 130 |
| 80 | 1 | 1 | 1 | 1 | |
| 90 | 1 | 1 | 1 | 1 | |
| 100 | 1 | 1 | 2 | 2 | |
| 110 | 21 | 3 | 39 | 34 | |
| 120 | 96 | 15 | alle > 130 | alle > 130 | |

Prüfung 2: WSF nach vier Wochen Lagerung der Klebstoffformulierung bei Raumtemperatur. Erst dann wurde die Hartfaserplatte mit Klebstoff beschichtet und die PVC-Folie nach Trocknung dagegen kaschiert.

Ablaufstrecke in mm bei der jeweiligen Prüftemperatur, Mittelwerte aus fünf Messungen

| Temperatur (°C) | Beispiel 1 | Beispiel 2 | Beispiel 3 (Vergleich) | Beispiel 4 (Vergleich) | Beispiel 5 (Vergleich) |
|---|---|---|---|---|---|
| | | | | | |
| 40 | - | - | - | - | - |
| 50 | 1 | 1 | 1 | 1 | 74 |
| 60 | 1 | 1 | 1 | 1 | alle > 130 |
| 70 | 1 | 1 | 1 | 1 | |
| 80 | 1 | 1 | 1 | 1 | |
| 90 | 1 | 1 | 1 | 2 | |
| 100 | 1 | 1 | 2 | 1 | |
| 110 | 10 | 5 | 11 | 11 | |
| 120 | 72 | 43 | 74 | 74 | |

In Prüfung 3 wurde die mit Klebstoff-Ansatz beschichtete Hartfaserplatte vier Wochen bei RT gelagert und dann erst die PVC-Folie aufkaschiert.

Ablaufstrecke in mm bei der jeweiligen Prüftemperatur, Mittelwerte aus fünf Messungen

| Temperatur (°C) | Beispiel 1 | Beispiel 2 | Beispiel 3 (Vergleich) | Beispiel 4 (Vergleich) | Beispiel 5 (Vergleich) |
|---|---|---|---|---|---|
| | | | | | |
| 40 | - | - | - | - | - |
| 50 | 2 | 3 | 2 | 2 | 19 |
| 60 | 1 | 5 | 1 | 1 | alle > 130 |
| 70 | 1 | 8 | 1 | 2 | |
| 80 | 2 | 9 | 2 | 5 | |
| 90 | 5 | 8 | 3 | 3 | |
| 100 | 18 | 3 | 24 | 1 | |
| 110 | 87 | 3 | 94 | 9 | |
| 120 | alle > 130 | 21 | alle > 130 | 82 | |

## Patentansprüche

1. Verfahren zur Verklebung von Substraten unter Verwendung eines Klebstoffs, der ein Polymer als Bindemittel und eine Carbodiimid-Gruppen enthaltende Verbindung I als Vernetzer enthält, **dadurch gekennzeichnet, dass** die Carbodiimid-Gruppen enthaltende Verbindung erhältlich ist durch Umsetzung von
a) Carbodiimiden des 1,3-Bis-(1 methyl-1-isocyanatoethyl) benzol, 1,4-Bis-(1methyl-1-isocyanatoethyl) benzol oder deren Gemische mit
b) Polyalkylenoxiden mit mindestens zwei gegenüber Isocyanat reaktiven Gruppen, vorzugsweise mindestens zwei Hydroxylgruppen und
c) gegebenenfalls weiteren mit a) oder b) reaktiven Verbindungen.

2. Verfahren gemäß Anspruch 1, **dadurch gekennzeichnet, dass** die Verbindung I zu mindestens 80 Gew. % aus a) und b) besteht.

3. Verfahren gemäß Anspruch 1 oder 2, **dadurch gekennzeichnet, dass** es sich bei b) um Polyalkylenoxide der Formel
H-O-(CH₂-CHR₁-O-)ₙ H
handelt, worin R 1 für H oder CH3 steht und n eine ganze Zahl von 1 bis 100 bedeutet.

4. Verfahren gemäß einem der Ansprüche 1 bis 3, **dadurch gekennzeichnet, dass** die Verbindungen I zu mehr als 40 Gew. % aus Ethylenoxideinheiten bestehen.

5. Verfahren gemäß einem der Ansprüche 1 bis 5, **dadurch gekennzeichnet, dass** es sich bei dem Polymer um ein Polyurethan, ein radikalisch polymerisiertes Polymer oder deren Gemische handelt.

6. Verfahren gemäß Anspruch 5, **dadurch gekennzeichnet, dass** das Polyurethan zu mindestens 60 Gew. % aus Diisocyanaten, Polyetherdiolen und/oder Polyesterdiolen aufgebaut ist.

7. Verfahren gemäß einem der Ansprüche 5 oder 6, **dadurch gekennzeichnet, dass** das Polyurethan einen Schmelzpunkt im Bereich von -50 bis 150°C, vorzugsweise von 0 bis 100°C hat.

8. Verfahren gemäß Anspruch 5, **dadurch gekennzeichnet, dass** das radikalisch polymerisierte Polymer zu mindestens 60 Gew. % aufgebaut ist aus sogenannten Hauptmonomeren, ausgewählt aus C1 bis C20 Alkyl(meth)acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden, Vinylethern von 1 bis 10 C Atome enthaltenden Alkoholen, aliphatischen Kohlenwasserstoffen mit 2 bis 8 C Atomen und ein oder zwei Doppelbindungen oder Mischungen dieser Monomeren.

9. Verfahren gemäß einem der Ansprüche 1 bis 8, **dadurch gekennzeichnet, dass** der Klebstoff 0,0001 bis 0,1 mol Carbodiimidgruppen auf 100 g Klebstoff (Wasser oder sonstige organische Lösemittel mit einem Siedepunkt unter 150°C bei 1 bar nicht mitberechnet) enthält.

10. Verfahren gemäß einem der Ansprüche 1 bis 9, **dadurch gekennzeichnet, dass** eine Polymerfolie, ein Holzfurnier oder Papier mit einem nicht-flexiblen Substrat verklebt wird.

11. Verfahren gemäß einem der Ansprüche 1 bis 10, **dadurch gekennzeichnet, dass** es sich bei dem nicht flexiblen Substrat um Teile aus Holz oder Kunststoff handelt.

12. Verfahren gemäß einem der Ansprüche 1 bis 10, **dadurch gekennzeichnet, dass** es sich bei dem nicht flexiblen Substrat um Teile aus gebundenen natürlichen oder synthetischen Fasern oder Spänen handelt, z. B. Hartfaserplatten, Mitteldichten Faserplatten (MDF-Platten).

13. Verfahren gemäß einem der Ansprüche 1 bis 12, **dadurch gekennzeichnet, dass** der Klebstoff auf ein flexibles Substrat aufgebracht wird, gegebenenfalls eine Trocknung zur Entfernung von Wasser oder organischen Lösemitteln erfolgt und die Klebstoffschicht bei der späteren Verklebung mit einem nicht flexiblen Substrat auf Temperaturen von 20 bis 200°C erwärmt wird.

14. Verfahren gemäß Anspruch 13, **dadurch gekennzeichnet, dass** das flexible Substrat nach Beschichtung mit dem Klebstoff und nach gegebenenfalls erfolgter Trocknung aufgewickelt und gelagert wird.

15. Flexible Substrate aus mindestens einem Träger und einer Klebstoffbeschichtung gemäß einem der Ansprüche 1 bis 9.

16. Verfahren gemäß einem der Ansprüche 1 bis 14, **dadurch gekennzeichnet, dass** das nicht flexible Substrat bei der Verklebung eine Temperatur von 50 bis 200°C aufweist.

17. Verklebte Verbunde, erhältlich durch ein Verfahren gemäß einem der Ansprüche 1 bis 14 oder Anspruch 16.

18. Carbodiimid-Gruppen enthaltende Verbindung 1, erhältlich durch Umsetzung von
a) Carbodiimiden des 1,3-Bis-(1 methyl-1-isocyanatoethyl) benzol, 1,4-Bis-(1 methyl-1-isocyanatoethyl) benzol oder deren Gemische mit
b) Polyalkylenoxiden mit mindestens zwei gegenüber Isocyanat reaktiven Gruppen, vorzugsweise mindestens zwei Hydroxylgruppen
c) gegebenenfalls weiteren mit a) oder b) reaktiven Verbindungen.

19. Wässrige Polymerdispersion, enthaltend eine Verbindung 1 gemäß Anspruch 18.

## Claims

1. A process for adhesively bonding substrates, using an adhesive comprising a polymer binder and a crosslinker compound I comprising carbodiimide groups, wherein the compound comprising carbodiimide groups is obtainable by reacting
a) carbodiimides of 1,3-bis(1-methyl-1-isocyanatoethyl)benzena, 1,4-bis(1-methyl-1-isocyanatoethyl)benzene or a mixture thereof with
b) polyalkylene oxides having at least two isocyanate-reactive groups, preferably at least two hydroxyl groups, and
c) if appropriate, further compounds reactive with a) or b).

2. The process according to claim 1, wherein at least 80% by weight of the compound I is composed of a) and b).

3. The process according to claim 1 or 2, wherein polyalkylene oxides b) are of the formula
H-O-(CH₂-CHR₁-O-)ₙ H
where R₁ is H or CH₃ and n is an integer from 1 to 100.

4. The process according to any one of claims 1 to 3, wherein more than 40% by weight of the compound I is composed of ethylene oxide units.

5. The process according to any one of claims 1 to 4, wherein the polymer is a polyurethane, a free-radically polymerized polymer or a mixture thereof.

6. The process according to claim 5, wherein the polyurethane is synthesized from at least 60% by weight of diisocyanates, polyetherdiols and/or polyesterdiols.

7. The process according to either of claims 5 and 6, wherein the polyurethane has a melting point in the range from -50 to 150°C, preferably from 0 to 100°C.

8. The process according to claim 5, wherein the free-radically polymerized polymer is synthesized from at least 60% by weight of principal monomers selected from C₁ to C₂₀ alkyl (meth)acrylates, vinyl esters of carboxylic acids comprising up to 20 carbon atoms, vinylaromatics having up to 20 carbon atoms, ethylenically unsaturated nitriles, vinyl halides, vinyl ethers of alcohols comprising 1 to 10 carbon atoms, aliphatic hydrocarbons having 2 to 8 carbon atoms and one or two double bonds, and mixtures of these monomers.

9. The process according to any one of claims 1 to 8, wherein the adhesive comprises 0.0001 to 0.1 mol of carbodiimide groups per 100 g of adhesive (excluding from the calculation water or other organic solvents having a boiling point below 150°C at 1 bar).

10. The process according to any one of claims 1 to 9, wherein a polymer sheet, a wood veneer or paper is bonded to a nonflexible substrate.

11. The process according to any one of claims 1 to 10, wherein the nonflexible substrate is a part made of wood or plastic.

12. The process according to any one of claims 1 to 10, wherein the nonflexible substrate is a part made of bound natural or synthetic fibers or chips, such as a hardboard or medium-density fiberboard (MDF) panel.

13. The process according to any one of claims 1 to 12, wherein the adhesive is applied to a flexible substrate, drying takes place if appropriate in order to remove water or organic solvents, and the adhesive layer, in the course of subsequent bonding to a nonflexible substrate, is heated at temperatures from 20 to 200°C.

14. The process according to claim 13, wherein the flexible substrate, after having been coated with the adhesive and, if appropriate, dried, is wound up and stored.

15. A flexible substrate comprising at least one support and an adhesive coating according to any one of claims 1 to 9.

16. The process according to any one of claims 1 to 14, wherein the nonflexible substrate in the course of bonding has a temperature from 50 to 200°C.

17. An adhesively bonded assembly obtainable by a process according to any one of claims 1 to 14 or claim 16.

18. A compound I comprising carbodiimide groups and obtainable by reacting
a) carbodiimides of 1,3-bis(1-methyl-1-isocyanatoethyl)benzene, 1,4-bis(1-methyl-1-isocyanatoethyl)benzene or a mixture thereof with
b) polyalkylene oxides having at least two isocyanate-reactive groups, preferably at least two hydroxyl groups, and
c) if appropriate, further compounds reactive with a) or b).

19. An aqueous polymer dispersion comprising a compound I according to claim 18.

## Revendications

1. Procédé pour effectuer le collage de substrats en utilisant une colle qui contient un polymère comme liant et un composé I contenant des groupes carbodiimide comme agent de réticulation, **caractérisé en ce que** le composé contenant des groupes carbodiimide peut être obtenu en faisant réagir :
a) des carbodiimides du 1,3-bis-(1-méthyl-1-isocyanatoéthyl)benzène, du 1,4-bis--f1-méthyl-1-isocyanatoéthyl)benzène ou leurs mélanges avec
b) des poly(oxydes d'alkylène) ayant au moins deux groupes réactifs vis-à-vis du groupe isocyanate, de préférence, au moins deux groupes hydroxyle, et
c) éventuellement, d'autres composés réactifs avec les composés a) ou b).

2. Procédé selon la revendication 1, **caractérisé en ce que** le composé I est constitué d'au moins 80 % en poids des composés a) et b)

3. Procédé selon la revendication 1 ou 2, **caractérisé en ce que** les composés b) sont représentés par des poly(oxydes d'alkylène) de formule suivante :
H-O- (CH₂- CHR₁-O-)ₙ H
dans laquelle R₁ représente H ou CH₃ et n représente un nombre entier de 1 à 100.

4. Procédé selon l'une quelconque des revendications 1 à 3, **caractérisé en ce que** les composés I sont constitués de plus de 40 % en poids d'unités oxyde d'éthylène.

5. Procédé selon l'une quelconque des revendications 1 à 4, **caractérisé en ce que** le polymère est représenté par un polyuréthanne, un polymère obtenu par polymérisation radicalaire ou leurs mélanges.

6. Procédé selon la revendication 5, **caractérisé en ce que** le polyuréthanne est constitué d'au moins 60 % en poids de diisocyanates, de polyétherdiols et/ou de polyesterdiols.

7. Procédé selon l'une quelconque des revendications 5 ou 6, **caractérisé en ce que** le polyuréthanne a un point de fusion situé dans la plage de températures de -50 à 150 °C, de préférence, de 0 à 100 °C.

8. Procédé selon la revendication 5, **caractérisé en ce que** le polymère obtenu par polymérisation radicalaire est constitué d'au moins 60 % en poids de ce que l'on appelle les monomères principaux, choisis parmi des (méth)acrylates d'alkyle en C1-C20, des esters vinyliques d'acides carboxyliques contenant jusqu'à 20 atomes de carbone, des composés vinylaromatiques contenant jusqu'à 20 atomes de carbone, des nitriles à insaturation éthylénique, des halogénures de vinyle, des éthers vinyliques d'alcools contenant 1 à 10 atomes de carbone, des hydrocarbures aliphatiques contenant 2 à 8 atomes de carbone et une ou deux doubles liaisons ou des mélanges de ces monomères.

9. Procédé selon l'une quelconque des revendications 1 à 8, **caractérisé en ce que** la colle contient 0,0001 à 0,1 mole de groupes carbodiimide sur 100 g de colle (eau ou autres solvants organiques ayant un point d'ébullition inférieur à 150 °C à une pression de 1 bar non compris).

10. Procédé selon l'une quelconque des revendications 1 à 9, **caractérisé en ce que** l'on colle une feuille polymère, un contreplaqué ou un papier à un substrat non flexible.

11. Procédé selon l'une quelconque des revendications 1 à 10, **caractérisé en ce que** le substrat non flexible est représenté par des parties en bois ou en matériau synthétique.

12. Procédé selon l'une quelconque des revendications 1 à 10, **caractérisé en ce que** le substrat non flexible est représenté par des parties constituées de fibres ou de copeaux naturels ou synthétiques agglomérés, par exemple, des panneaux de fibres rigides, des panneaux de fibres de densité moyenne (panneaux MDF).

13. Procédé selon l'une quelconque des revendications 1 à 12, **caractérisé en ce que** l'on applique la colle sur un substrat flexible, que l'on effectue, éventuellement, un séchage pour éliminer l'eau ou les solvants organiques et que l'on chauffe la couche de colle, lors de l'étape suivante de collage avec un substrat non flexible, à des températures de 20 à 200 °C.

14. Procédé selon la revendication 13, **caractérisé en ce que** le substrat flexible est enroulé et stocké après qu'il a été revêtu avec l'adhésif et qu'il a éventuellement subi un séchage.

15. Substrats flexibles constitués d'au moins un support et d'un revêtement adhésif selon l'une quelconque des revendications 1 à 9.

16. Procédé selon l'une quelconque des revendications 1 à 14, **caractérisé en ce que** le substrat non flexible présente, lors du collage, une température de 50 à 200 °C.

17. Composites collés, que l'on peut obtenir par un procédé selon l'une quelconque des revendications 1 à 14 ou selon la revendication 16.

18. Composé I contenant des groupes carbodiimide, que l'on peut obtenir en faisant réagir:
a) des carbodiimides du 1,3-bis-(1-méthyl-1-isocyanatoéthyl)benzène, du 1,4-bis-(1-méthyl-1-isocyanatoéthyl)benzène ou leurs mélanges avec
b) des poly(oxydes d'alkylène) ayant au moins deux groupes réactifs vis-à-vis du groupe isocyanate, de préférence, au moins deux groupes hydroxyle
c) éventuellement, d'autres composés réactifs avec les composés a) ou b).

19. Dispersion polymère aqueuse, contenant un composé I selon la revendication 18.
